# EUROPEAN PATENT SPECIFICATION

(11) **EP 1 502 113 B1**
(45) Date of publication and mention of the grant of the patent: **12.08.2009**
(21) Application number: 03749868.0
(22) Date of filing: 06.05.2003
(51) Int. Cl.: G01N 33/574

(54) **A NOVEL BIOLOGICAL CANCER MARKER AND METHODS FOR DETERMINING THE CANCEROUS OR NON-CANCEROUS PHENOTYPE OF CELLS**
EIN NEUER BIOLOGISCHER TUMORMARKER UND METHODEN FÜR DIE DETEKTION DES KREBSARTIGEN ODER NICHT KREBSARTIGEN PHENOTYPS VON ZELLEN
NOUVEAU MARQUEUR BIOLOGIQUE CANCEREUX ET PROCEDES DE DETERMINATION DU PHENOTYPE CELLULAIRE CANCEREUX OU NON CANCEREUX

(30) Priority: 07.05.2002 EP 02291166
(43) Date of publication of application: 02.02.2005
(73) Proprietor: Centre National de La Recherche Scientifique-CNRS, 75794 Paris Cedex 16 (FR)
(72) Inventor: ROUX, Pierre, F-34980 Saint Gely du Fesc (FR); GADEA, Gilles, F-34000 Montpellier (FR)
(74) Representative: Catherine, Alain
(86) International application number: PCT/EP2003/004727
(87) International publication number: WO 2003/096019

(56) References cited:
- NICHOLSON N B ET AL: "F ACTIN PATTERNS QUANTITATED WITH FL PHALLOIDIN IN SKIN FIBROBLASTS OF INDIVIDUALS GENETICALLY PREDISPOSED TO COLON CANCER" SCHWEIGER, H. G. (ED.). INTERNATIONAL CELL BIOLOGY 1980-1981;2ND, 1981, pages P331-335, XP001098259 XVIII+1033P. SPRINGER-VERLAG: BERLIN, WEST GERMANY; NEW YORK, N.Y., USA. ILLUS. ISBN 3-540-10475-5; ISBN 0-387-10475-5. 1981
- RAO J YU ET AL: "CELLULAR F-ACTIN LEVELS AS A MARKER FOR CELLULAR TRANSFORMATION CORRELATION WITH BLADDER CANCER RISK" CANCER RESEARCH, vol. 51, no. 11, 1991, pages 2762-2767, XP001093920 ISSN: 0008-5472
- KARASAKI S ET AL: "SURFACE MORPHOLOGY AND NUCLEOSIDE PHOSPHATASE ACTIVITY OF RAT LIVER EPITHELIAL CELLS DURING ONCOGENIC TRANSFORMATION IN-VITRO" CANCER RESEARCH, vol. 37, no. 10, 1977, pages 3516-3525, XP001098272 EN ISSN: 0008-5472
- DATABASE BIOSIS [Online] BIOSCIENCES INFORMATION SERVICE, PHILADELPHIA, PA, US; 2001 SHEKARABI M ET AL: "DCC promotes filopodia and lamellipodia formation via mechanisms that require and activate Cdc42 and Rac1." Database accession no. PREV200100541805 XP002209258 & SOCIETY FOR NEUROSCIENCE ABSTRACTS, vol. 27, no. 2, 2001, page 1540 31st Annual Meeting of the Society for Neuroscience;San Diego, California, USA; November 10-15, 2001 ISSN: 0190-5295
- GADEA GILLES ET AL: "Regulation of Cdc42-mediated morphological effects: A novel function for p53." EMBO (EUROPEAN MOLECULAR BIOLOGY ORGANIZATION) JOURNAL, vol. 21, no. 10, 15 May 2002 (2002-05-15), pages 2373-2382, XP001093930 May 15, 2002 ISSN: 0261-4189

## Description

### FIELD OF THE INVENTION

The present invention relates to newly developed assays for detecting, diagnosing, monitoring, staging cancer cells and designing anticancerous treatments, particularly for gastro-intestinal cancers.

### BACKGROUND OF THE INVENTION

A major problem in the treatment of cancer remains the lack of availability of reproducible and easy-to-use detection means.

A lot of prior art cancer assays are based on the detection of various biological markers including proteins or glycosylated proteins which are specifically expressed in cancer cells.

For example, clinical staging of prostate cancer generally depends on the results of three tests that are performed in the following order: a PSA (prostate-specific antigen) blood test as a screening method; DRE (digital rectal examination) for an initial indication of palpable disease; and a biopsy to obtain samples for histological examination.

Down-regulated genes such as tumors suppressors, invasion suppressors or metastasis suppressors may be used as prostate cancer markers, such as KAI1.

Breast cancer is the most diagnosed cancer in women and the second leading cancer related cause of death in women. Cancer assays for detecting mammary gland cancer include detection of mutations in the BRCA1 gene. However, less than 10 percent of mammary gland cancer cases are thought to be related to the BRCA1 gene.

Cancer of the colon is the second most frequently diagnosed malignancy in the United States, as well as the second most common cause of cancer death. Colon cancer is a highly treatable and often curable disease when localized in the bowel.

Due to its proximity, cancer of the colon often metastasised to the small intestine. The prognosis of the cancer spreading to the small intestine is related to the degree of penetration of the tumor through the bowel wall and the presence or absence of nodal involvement.

Various characteristics also assist in prognosticating colon cancer and its spread to the small intestine. For example, bowel obstruction and bowel perforation are indicators of poor prognosis. Elevated pretreatment serum levels of carcinoembryonic antigen (CEA) and of carbohydrate antigen 19-9 (CA 19-9) also have a negative prognostic significance.

Because of the frequency of these types of cancer, the identification of high risk groups, the demonstrated slow growth of primary lesions and the better survival of early-stage lesions, screening for gastro-instestinal cancers should be a part of routine care for all adults starting at age 50, especially those with first-degree relatives with colorectal cancer.

Procedures used for detecting, diagnosing, monitoring, and staging cancer of the colon, small intestine or stomach are of critical importance to the outcome of the patient. Patients diagnosed with early stage cancer generally have a much greater five years survival rate as compared to the survival rate for patients diagnosed with distant metastasised cancers.

New diagnostic methods which are more sensitive and specific for detecting cancer of the stomach, small intestine and colon are clearly needed.

However, despite the discovery of various markers of colon carcinoma, such as CSG1-13, Cln114 (galectin-4) and Cln115 (human carbonic anhydrase I), the availability of cancer assays ensuring a sufficient degree of confidence in the prognostic results has not yet been reached in the art.

To date, the sole biological marker which is conventionally used as a marker for colon carcinoma is the carcinoembryonic antigen (CEA). However, the serum titration of CEA does not allow the early detection and diagnosis of colon carcinoma and cannot be used to predict or in contrast to exclude the presence of metastasis, notably hepatic metastasis.

Similarly, the detection of mutations which alter the tumor suppressor p53 gene cannot be used as sufficiently predictive or diagnostic markers of cancers. On the one hand, mutation in the p53 gene only appears at a late stage of the tumoral progression. On the other hand, an unaltered p53 gene sequence may be found In tumor cells having a reduced expression level of the p53 protein, for example due to an alteration of one or several of the activation pathways of the p53 proteins.

Several prior art studies relate to the cell surface morphology during oncogenesis.

Nicholson et al. (1981, International cell biology, pages 331-335) relates to the study of intracellular F-actin in skin fibroblasts of individuals genetically predisposed to colon cancer. This document discloses quantification methods of F-actin-containing structures on fixed and stained cells.

Similarly, Rao et al. (1991, Cancer Research, Vol. 51(11); pp 2762-2767) discloses a method for detecting the content in F-actin which involves the preliminary fixation of cells and triple labelling.

Karasaki et al. (1997, Cancer Research, Vol. 37(10), pp 3516-3525) relates to a study of the surface morphology of rat liver epithelial cells during oncogenic transformation. This article discloses a method for cell analysis comprising a preliminary step of cell fixation with glutaraldehyde.

Shekarabi et al. (2001, Society for Neuroscience abstracts, Vol. 27(2), page 1540) consists of a general disclosure relating to the presence of filprodia at the cell surface.

Thus, there is a need in the art for novel biological markers of cancers, notable gastrointestinal cancers including stomach, small intestine and colon cancers, as well as for methods for predicting, detecting, diagnosing, staging and monitoring cancer with a high degree of certainty.

Further, there is a need in the art for methods allowing the detection of the metastatic potential of a tumor cell, notably in order to adapt the cancer treatment to the phenotype stage of cells collected from a patient.

Additionally, the availability of such a method would also allow the one killed in the art to carry out a screening for carcinogenic or in contrast anti-cancerous compounds.

### SUMMARY OF THE INVENTION

The present invention relates to the use of the frequency value of filopodia generation in a sample cell population as a biological marker for determining the cancerous, metastatic cancerous or normal phenotype of said cell population, wherein the frequency of filopodia generation consists of counting the number of filopodia formed at successive instants during a predetermined period of time

It also relates to a method for determining *in vitro* the cancerous, metastatic cancerous or normal phenotype of cells contained in a sample, wherein said method comprises a step of *in vitro* measuring the frequency of filopodia generation in the cells contained in the sample wherein the frequency of filopodia generation consists of counting the number of filopodia formed at successive instants during a predetermined period of time

The present invention further concerns in vitro methods for the screening of the carcinogenic properties of a compound or of the metastatic properties, of a compound which make use of the general method described above.

This invention also concerns in vitro methods for the screening of the anti-carcinogenic or of the anti-metastatic properties of a compound which make use of the general method described above.

The invention also relates to a method for designing an anti-cancerous therapeutic treatment of a cancerous patient which comprises the step of determining the cancerous state of cells contained in a sample derived from a primary tumor of said patient by carrying out one of the methods described above.

### BRIEF DESCRIPTION OF THE FIGURES

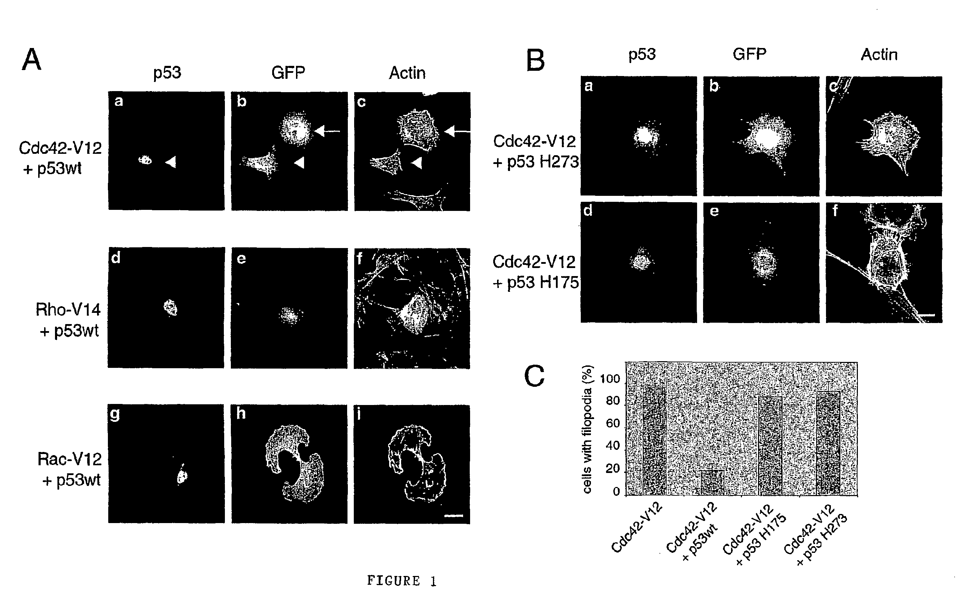

**Figure 1** **: Effects of p53 on Cdc42-, Rac1- and RhoA-induced F-actin changes.**
   A: Proliferating MEF were co-transfected with plasmids encoding wt p53 and GFP-tagged Cdc42-V12 (panels a, b and c), RhoA-V14 (panels d, e, and f) or Rac1-V12 (g, h and i). 20-24 hours later, cells were fixed and stained for F-actin (panels c, f and i), and p53 using 240 monoclonal antibody (panels b, e and h). GFP positive cells were also detected (panels a, d and g). Bar scare : 10 µm.
   B: MEF were transfected with GFP-tagged Cdc42-V12, in combination with dominant negative p53 mutants (p53 H273 in panels a, b and c or p53H175 in panels d, e and f). Cells were treated as in Figure 1A : co-transfected cells were visualized for p53 (panels a and b), GFP (panels b and e) and F-actin (panels c and f). Bar scale : 10 µm.
   C: Quantification of MEF having filopodia. MEF, treated as in figures 1A and 1B, were scored positive when presenting at least 5 filopodia. For each experiment 300 cells were counted. The mean average and SD were calculated from 6 independent experiments.

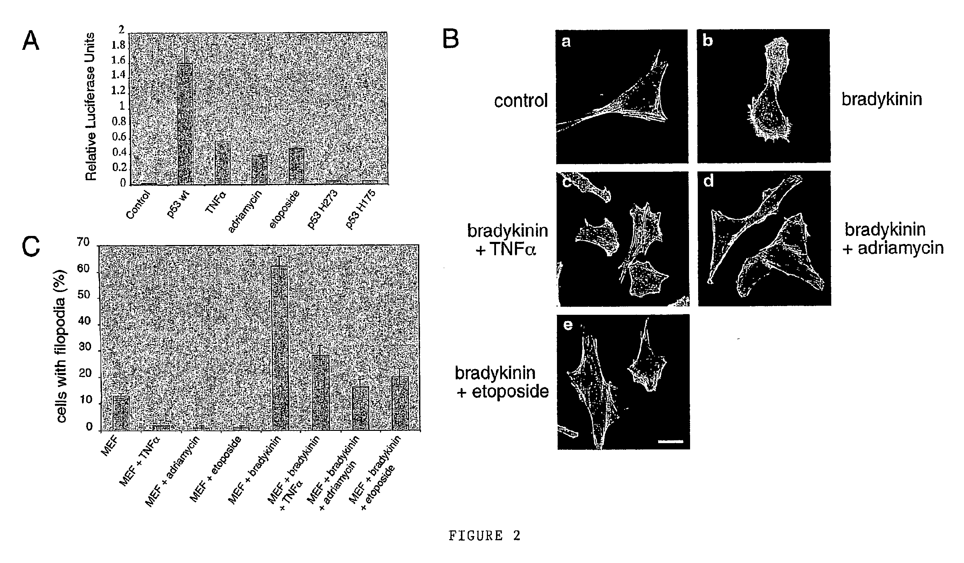

**Figure 2****: Endogenous Cdc42-induced filopodia formation is inhibited by TNFa-stimulated p53 activity.**
   A: MEF were transfected with the firefly luciferase gene under the control of the mdm2 promoter and treated for 10 hours either with TNFa adriamycin, or etoposide. The luciferase activity was assayed 24 hours after transfection. The p53 responsive element construct was transfected in combination with either p53wt, as a positive control, or with the dominant negative mutants p53-H273 or p53-H175. The luciferase activity was assayed 24 hours later. The results are shown as mean +/S.D. of four measurements.
   B: Proliferating MEF were either untreated (panel a), treated with bradykinin alone (panel b), or pretreated with respectively TNFa (panel c) adriamycin (panel d) or etoposide (panel e) for 10 hours, followed by a 12 minutes treatment with bradykinin, then stained for F-actin. Bar scale : 10 µm.
   C: Quantification of MEF having filopodia. MEF, treated as in figure 2B were scored positively when presenting at least 5 filopodia. For each experiment, 100 cells were scored and results are the mean +/- SD of 3 independent experiments.

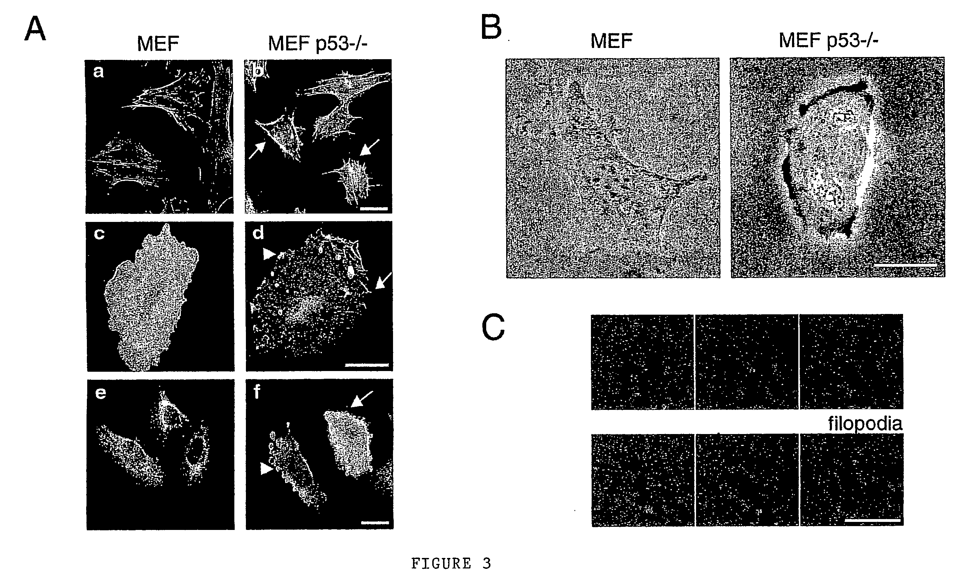

**Figure 3****: Absence of p53 activity leads to a persistent accumulation of filopodia.**
   A: MEF (panels a, c and e) and MEF p53-/- (panels b, d and f) were stained for F-actin (panels a and b) or processed for SEM (panels c and d), or analysed for ezrin distribution (panels e and f). Bar scale : 10 µm.
   B: Still phase contrast images issued from the videos of MEF (panel a) and MEF p53 -/- (panel b) are shown. Bar scale : 10 µm.
   C: Detailed phase contrast images describe the sequential initiating steps of filopodia formation in MEF p53 -/- : cytoplasm engorgement (E), focal densities (fd), nubs and filopodia. Bar scale : 1 µm.

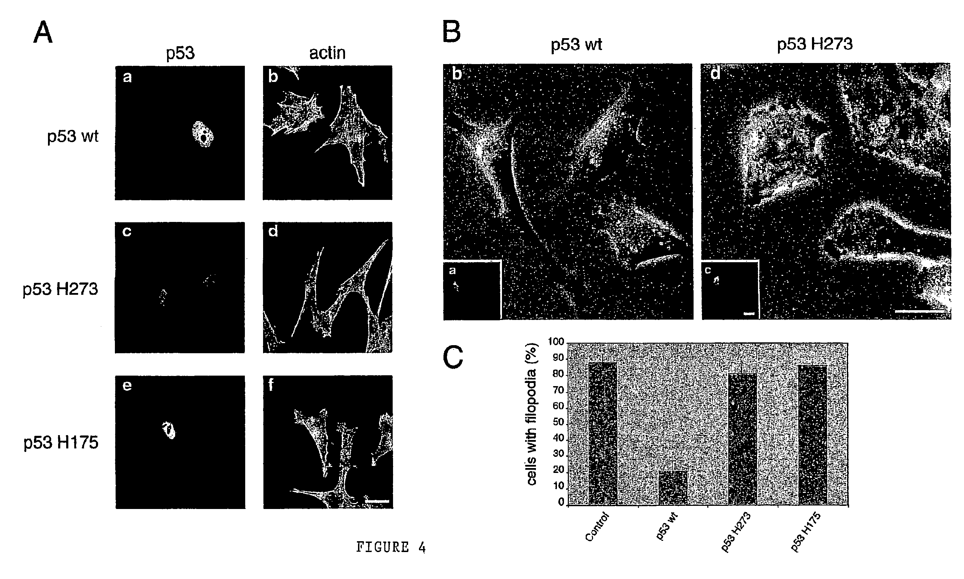

**Figure 4** **: p53 wt is required to inhibit filopodia.**
   A: MEF p53-/- were transfected with plasmids expressing either p53 wt (panels a and b), p53 H273 (panels c and d) or p53 H175 (panels e and f). Cells were stained for p53 expression (panels a, c and e) and F-actin (panels b, d and f). Bar scale : 10 µm.
   B: MEF p53-/- transfected with GFP-tagged forms of either p53 wt (panels a and b) or p53 H273 (panels c and d), were detected for GFP (panels a and c). Still phase contrast images issued from the videos shows cell transfected with either p53 (panel b) or p53 H273 (panel d). Bar scale : 10 µm.
   C: Quantification of MEF presenting filopodia. MEF, treated as in figure 4B, were scored positively when presenting at least 5 filopodia. For each experiment, 50 cells were scored and results presented are the mean +/- SD of 3 independent experiments.

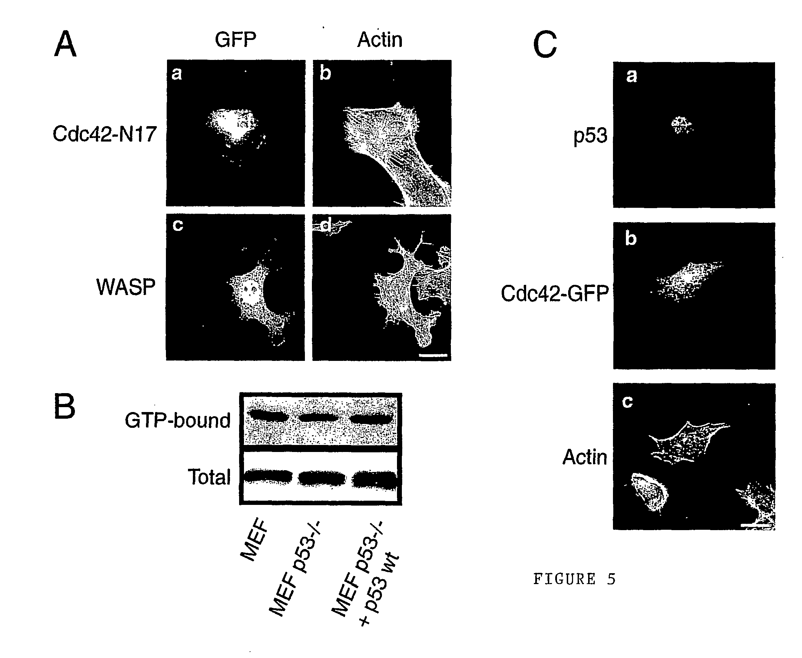

**Figure 5****: p53 acts downstream of Cdc42 to modify actin cytoskeleton.**
   A: MEF p53-/- were transfected with plasmids encoding GFP-tagged Cdc42-N17 (panels a and b) or MYC-tagged CRIB domain of WASP (panels c and d). Transfected cells detected for GFP (panel a) or stained for MYC (panel c), were stained for F-actin (panels b and d). Bar scale : 10 µm.
   B: MEF and MEF p53-/- transfected or not with p53 wt were lysed and the GTP-bound form of Cdc42 was analysed as described in Materials and Methods. Cdc42-GTP precipited with GST-PAK1 and total Cdc42 present in the lysates was analysed by immunoblotting with an anti-Cdc42 antibody.
   C: MEF p53 -/- were cotransfected with MYC-tagged p53 wt and GFP-tagged Cdc42-V12 (panels a, b and c). Fixed cells were visualized for GFP (panel b) and stained for p53 (panel a) and actin (panel c). Bar scale : 10 µm.

### DETAILED DESCRIPTION OF THE INVENTION

Surprisingly, it has been found according to the invention that the phenotype of a normal non-cancerous cell, of a cancerous cell and of a metastatic cancerous cell could be easily assessed by measuring the frequency value of filopodia generation in a sample cell population.

Most classical morphological characteristics of a cell include membrane ruffling, spreading properties, adhesion properties, stress fibers, lamellipodia and membrane filopodia.

Membrane filopodia are specific actin-containing structures protruding from the cell surface.

Membrane filopodia consist of long F-actin rich membrane extensions which may be found in numerous cell types including normal cells such as T-lymphocytes, oligodendrocytes or several immature cells from the CNS, as well as cancer cells.

It has now been shown according to the invention that there exists a clear causal effect between an alteration of the expression of the tumor suppressor p53 protein in normal cells and a dynamic activation of filopodia formation in these cells.

This dynamic filopodia formation in cells wherein has been experimentally mimicked a cancerous phenotype can exclusively be detected by assessing the number of membrane filopodia formed during a determined period of time in each cell of the cell population assayed.

Further, it has been shown according to the invention that the measure of the number of membrane filopodia formed in each cell of a cell population during a predetermined period of time provides a highly descriminant marker allowing to distinguish (i) between normal non-cancerous cells and cancerous cells, on the one hand and (ii) between cancerous non-metastatic cells and cancerous metastatic cells, on the other hand.

Additionally, it is also shown that this new cancer marker can be used irrespective of the type of cancer under study, since it is useful for detecting the presence of different kind of cancer cells, such as for example carcinoma cells and adenocarcinoma cells.

Thus, a first object of the invention consists of the use of the frequency value of filopodia generation in a sample cell population as a biological marker for determining the cancerous, metastatic cancerous or normal phenotype of said cell population, wherein the frequency of filopodia generation consists of counting the number of filopodia formed at successive instants during a predetermined period of time.

The present invention further relates to a method for determining *In vitro* the cancerous, metastatic cancerous or normal phenotype of cells contained in a sample, wherein said method comprises a step of *in vitro* measuring the frequency of filopodia generation or formation in the cells contained in the sample, wherein the frequency of filopodia generation consists of counting the number of filopodia formed at successive instants during a predetermined period of time.

Because filopodia are structures that are particularly dynamic and because their presence at the cell surface is transient and rapid, the sole' static numbering of mean filopodia structures in a cell population at a given instant would not have allowed the one skilled in the art to discriminate between populations of cancerous and non-cancerous cells.

For the purpose of the method above, the frequency of filopodia generation or formation is assessed by counting the number of membrane filopodia successively formed at the cell surface during a predetermined period of time.

Preferably, the frequency of filopodia generation or formation is calculated by counting the number of filopodia protruding out of one chosen cell in a constant unit of area and time.

Most preferably, the frequency of filopodia formation is calculated as the mean frequency of at least 10 cells, advantageously at least 50 cells, and more preferably at least 100 cells contained in the examined sample.

The number of cells studied for calculating the frequency of filopodia formation in a given cell population might be outside the cell ranges specified above, although less convenient.

According to a first specific aspect of the method, the constant unit of area which is analysed for each cell is comprised between 0.0001 µm² and 0.01 µm², preferably between 0.0005 and 0.005 µm².

Indeed, the constant unit of area analysed for calculating the frequency of filopodia formation may be outside the area ranges specified above, although less convenient.

In a second specific aspect of the method, the constant time period during which the frequency of filopodia formation is analysed in one chosen cell is comprised between 0.1 and 10 min., preferably between 0.5 and 5 min. and most preferably between 0.7 and 3 min, for example 1 min.

Indeed, the predetermined time period during which the frequency of filopodia formation is calculated may be outside the time period ranges specified above, although less convenient.

The frequency of filopodia generation or formation in the cells contained in the sample is preferably expressed as the mean number of filopodia formed per constant period of time and for a constant unit of area of cell, for at least 10 cells.

Illustratively, the frequency of filopodia generation or formation is calculated using a microscope, preferably a phase-contrast microscope, equipped with an automatic shutter and an appropriate camera, for example a CCD camera connected with a computer loaded with a software permitting the compilation of the collection of successive pictures of the cell surface area studied, each picture corresponding to an instant of the predetermined period of time during which the assay is carried out.

The compilation of the collection of successive pictures so captured allows the dynamic counting of filopodia formation in the cell area studied during the entire predetermined period of time of the assay, as disclosed in the examples.

Indeed, any other appropriate device, installation or equipment allowing the assessment of the frequency of filopodia generation or formation may also be used to carry out the method described above.

The sample containing the cells which are assayed may be of various kinds.

For the purpose of determining the cancerous, eventually metastatic cancerous, or normal phenotype of a cell population from a patient, said sample may be a sample derived from blood and containing the cells of interest as regards the type of cancer to be diagnosed, for example lymphocytes or polymorphonuclear cells. Said sample may also consist in cells derived from a biopsy collection from the patient and then cultured in an appropriate culture medium.

Such a biopsy may be for example a tissue biopsy from colon, small intesine or stomach.

Single tumor cells could be isolated from tissue biopsy by trypsinization (Trypsin 0.5%, EDTA, 0.1 mM in PBS buffer) to establish tumor primary cultures, as previously described (Trojanek et al., 2000, Cancer Biother. Radiopharm. 15(2): 169-74; Wilson et al, 1987, Cancer Research, 47 (10) 2704-13).

As a preferred embodiment of the method, the cells which are analysed for the purpose of calculating the frequency of filopodia generation or formation are grown in appropriate culture conditions allowing their viability and their normal growth during the assay.

More preferably, the cell population contained in the sample is grown in an appropriate culture medium, appropriate temperature conditions and appropriate moisture and gas composition conditions in order to avoid any alteration of their physiology, and thus their capacity of forming filopodia, during the whole assay.

Illustratively, the cells contained in the sample are cultured in the appropriate culture medium at 37°C and in a wet, 5% CO₂ atmosphere.

In a first specific embodiment of the method above, said method is used for determining *in vitro* the cancerous or normal phenotype of cells contained in a sample and comprises the steps of :
a) measuring the frequency of filopodia generation or formation in the cells contained in the sample;
b) comparing the frequency measured in step a) with the expected frequency of filopodia generation in (i) a control sample containing normal non-cancerous cells or (ii) a control sample containing tumor cells.

Preferably, the frequency comparison carried out in step b) of the method above is performed by using the expected frequency of filopodia generation in control samples containing cells, either non-cancerous cells or normal cells, of the same type as that of the cell population studied.

For example, when assessing the cancerous or non-cancerous state phenotype of a suspected melanoma or leukaemia cell containing sample under study, the comparison carried out in step b) is most preferably performed with the expected frequency of filopodia generation in control samples containing normal or in contrast cancerous melanocytes or lymphocytes, respectively.

As another illustrative example, when the cell sample studied originates from a patient suspected to be affected with a colorectal cancer, the comparison carried out in step b) is preferably performed by using the expected frequency of filopodia generation in control samples containing either normal or in contrast cancerous cells of the epithelial type, most preferably from colon epithelial.

The control frequency values of filopodia generation used for the comparison of step b) of the method above are generally predetermined.

For example, the experimental results of the examples show that a frequency value of filopodia formation of less than 0.1 filopodia, min⁻¹/µm² found in the cell sample under study means that the cells are non-tumor cells. Similar expected frequency for normal cells have been calculated for both fibroblast and epithelial cells.

Further, it has been shown that a frequency value of filopodia generation of more than 0.1 filopodia.min⁻¹/µm² means that the cell population under study has a cancerous phenotype.

Indeed, the discrimination potential of the method between cancerous and non-cancerous cells will have an increased predictive or diagnostic accuracy if the comparison carried out in step b) is systematically performed against the expected frequency of filopodia generation in control samples of the same type as that of the cells being assayed.

The determination of threshold frequency values for each cell type of interest, in order to carry out the method above with a maximum degree of confidence, is solely a matter of routine work for the one skilled in the art.

Further, it has been shown that the method according to the invention also allows to discriminate between cancerous and metastatic cancerous cells. This discrimination potential of the method according to the invention is highly important since it will greatly influence the type of therapeutic treatment which may be efficient for the patient.

For example, due to its proximity, cancer of the colon often metastasises to the small intestine, which is a major therapeutic problem and often is the ultimate cause of death. It is thus a highly concern to discriminate between cancerous and metastatic cancerous cells in a sample originating from a cancer patient, notably in order to adapt the therapeutic treatment after the cell staging determination.

It has been shown in the examples that the frequency value of filopodia generation in cancerous non-metastatic cells is comprised between 0.1 and 0.5 filopodia.min⁻¹/µm², and often between 0.2 and 0.5 filopodia.min⁻¹/µm². In contrast, the frequency value of filopodia generation in metastatic tumor cells is superior to 0.5 filopodia.min-¹/µm². This frequency threshold values have been obtained for carcinoma and adenocarcinoma of epithelial cells, respectively.

Indeed, as for the determination of the threshold frequency values for discriminating cancerous cells and normal non cancerous cells, the threshold frequency values permitting the discrimination between metastatic and non-metastatic cancerous cells should preferably be predetermined for a given cell type in order to increase the accuracy of the method, which is a matter of routine work for the one skilled in the art.

Thus, in a second specific embodiment of the method according to the invention, said method is used for determining *in vitro* the metastatic cancerous or the non-metastatic cancerous phenotype of cells contained in a sample, and comprises the steps of :
a) measuring the frequency of filopodia generation or formation in the cells contained in the sample;
b) comparing the frequency measured in step a) with the expected frequency of filopodia generation in (i) a control sample containing cancerous non-metastatic cells or (ii) a control sample containing metastatic tumor cells.

As already disclosed above, the frequency of filopodia generation in the method according to the invention, in every of its specific embodiments, is preferably measured by microscopy.

Since the method of the invention allows the discrimination between normal cells and cancerous cells, on the one hand, and between metastatic and non-metastatic cancerous cells, on the other hand, it will be easily implemented for screening various compounds for their carcinogenic, metastatic or in constrast anti-carcinogenic or anti-metastatic properties.

Methods for the screening of the carcinogenic properties of a given compounds are particularly useful in the whole field of industry, wherein sanitary requirements as regards the work environmental conditions are increasingly drastic and wherein is increasingly avoided any contact of the worker with toxic substances. These screening methods are also of a high usefulness in the food, in the cosmetics and in the pharmaceutical industry wherein the toxicity of every active principle, excipient or additive, and specifically their neoplastic properties, are to be rigorously assessed before the marketing of new foods, cosmetic compositions or pharmaceutical formulations.

Another object of the invention consists of a method for the in vitro screening of the carcinogenic properties of a compound, wherein said method comprises the steps of :
a) incubating cells having a normal non-cancerous phenotype with said compound;
b) detecting the conversion of the normal cells into tumor cells phenotype by carrying out the method for determining *in vitro* the cancerous, metastatic cancerous or normal phenotype of cells that is described in detailed above.

A further object of the invention consists of a method for the in vitro screening of the metastatic properties of a compound, wherein said method comprises the steps of :
a) incubating cells having a non-metastatic tumor phenotype with said compound;
b) detecting the conversion of the tumor cells into metastatic tumor phenotype by carrying out the method for determining *in vitro* the cancerous, metastatic cancerous or normal phenotype of cells which is disclosed in detail above.

One of the technical advantages of the two screening methods above lies in the physiological conditions wherein these methods are carried out, which actually mimick the *in vivo* action of the tested compound in a host organism, specifically a mammal, either a human or' a non-human mammal.

Additionally, methods for the screening of the anticarcinogenic or antimetastatic properties of a compound are highly useful in the medical field, specifically in finding active principles that may be used for manufacturing drugs against cancer.

The present invention has further as an object a method for the in vitro screening of the anti-carcinogenic properties of a compound, wherein said method comprises the steps of:
a) incubating cells having a tumor cell phenotype with said compound;
b) detecting the conversion of the tumor cells into non-tumor cell phenotype by carrying out the method for determining *in vitro* the cancerous, metastatic cancerous or normal phenotype of cells which is disclosed in detailed above.

The invention also relates to a method for the in vitro screening of the anti-metastatic properties of a compound, wherein said method comprises the steps of :
a) incubating cells having metastatic tumor cell phenotype with said compound;
b) detecting the conversion of the metastatic tumor cells into non-metastatic tumor cell phenotype or into non-tumor cell phenotype by carrying out the method for determining *in vitro* the cancerous, Metastatic cancerous or normal phenotype of cells described above.

As used herein, the term "incubating cells" means bringing into contact the cells growing in optimal culture conditions with the compound to be tested. Most preferably, a serial of distinct cell cultures are brought into contact with increasing concentrations of the compound to be tested in order to assess not solely its properties but also the concentration value of this compound upon which said compound exhibit the properties tested. Most preferably, the serial of distinct cell cultures comprises control cells which are not brought into contact with the compound to be tested. Eventually, one or several cell cultures are brought into contact with a concentration of respectively one or several compounds of known cancerous or anti-cancerous properties, at a concentration wherein said compound(s) are known to exhibit said cancerous or anti-cancerous properties.

As used herein, "cell culture" means a sample wherein the cells are cultured on a substrate, which may consist of any culture substrate known in the art, such as Petri dishes or plastic cell culture microplates conventionally used by the one skilled in the art.

Most preferably, the "cell culture" also comprises means that will ensure the control of the moisture, gas composition and temperature conditions while carrying out any specific embodiment of the methods according to the invention.

Indeed, the method according to the invention is very useful for the physician in order to adapt the nature, the composition or the dose of anti cancer therapeutic treatment to be administered to a specific patient. By determining the cancerous state of a cell population originating from a biopsy performed on a patient to be treated, the method of the invention will provide information necessary to orientate the practioner towards a more or less aggressive therapeutic treatment.

For example, if, by performing the method according to the invention, it is stated that the patient cells are cancerous but not metastatic, a therapeutic treatment with low amounts of anti-cancer drug(s) will be sufficient and will avoid numerous undesirable side effects that would be enhanced if higher amounts of drug(s) would have been administered to said patient.

In contrast, if, by performing the method according to the invention, it is stated that the patient cells originating from a biopsy are metastatis cells, the therapeutic treatment may be adapted by administering higher amount of anti-cancer active principles or alternatively distinct and more efficient active principles.

Another object of the present invention consists of a method for designing an anti-cancerous therapeutic treatment of a cancerous patient comprising the step of determining the cancerous state (phenotype) of cells contained in a sample derived from a primary tumor of said patient by carrying out the method for determining *in vitro* the cancerous, metastatic cancerous or normal phenotype of cells which is described in detail above.

The present invention will now be further illustrated, without being limited to, the examples below.

### EXAMPLES:

### MATERIAL AND METHODS FOR EXAMPLES 1 TO 4

### DNA constructs and reagents.

Human wild type p53 cDNA or its mutated forms, H175 and H273 were cloned into the BamH1 site of pCDNA3 vector (Invitrogen), then transferred in pEGFPC1 (Clontech) to give GFP-tagged proteins. Constructs expressing MYC epitope-tagged mutant Rac1, Cdc42 and RhoA proteins and their various mutants were kindly provided by P. Chavrier (Dutartre et al., 1996). The GFP fusion proteins were cloned in the pEGFP-C1 vector (Gauthier-Rouviere et al., 1998), (Ory et al., 2000), (Roux et al., 1997). The pcDNA3myc-NWASP containing the Cdc42-interacting domain of WASP was previously described in (Philips et al., 2000). The pGL2B-mdm2 plasmid in which the reporter gene luciferase is controlled by the p53-responsive element of mdm2 (Barak et al., 1994) was a kind gift of E. Yonish-Rouach. pTKRL plasmid was from Promega. TNFα, adriamycin and etoposide (Sigma) were used in all experiments at the concentration of 100 ng.ml⁻¹, 5 µg.ml⁻¹ and 25 µg.ml⁻¹, respectively.

### Cell culture, transfection.

Homozygous (p53 -/-) were originally generated in mating heterozygote (p53 +/-) mice of C57BL/6 and 129/Sv genetic background (Jacks et al., 1994), and were obtained from C.D.T.A. (Orleans, France), then were maintained on a 129/Sv x C57BU6 genetic background. MEF were generated from individual fetuses isolated from pregnant C57BL/6 females at 12 days previously mated with 129/Sv males to respect genetic background. MEF, MEF p53-/- were cultured at 37°C in the presence of 5% CO₂ in DMEM medium supplemented with 10 % fetal calf serum (FCS). For immunofluorescence experiments, cells were plated on 18 mm diameter glass coverslips 16-24 hours before transfection by the lipofectamine method (0.5 to 1 µg of plasmid DNA per 35 mm diameter well containing 3 glass coverslips), as recommended by the supplier (Gibco-BRL). Four hours after the transfection, the medium was replaced by DMEM supplemented with 10% FCS. Expressing cells were observed under fluorescence microscope 18 to 24 hours after transfection.

### Immunofluorescence and filopodia measurements.

MEM and MEF p53-/- were transfected on coverslips at the confluence of approximately 30 %. 20-24 hours later, cells were fixed for 5 min in 3.7% formalin (in PBS) followed by a 15 min permeabilization with 0.1% Triton-X100 (in PBS) and incubation in PBS containing 0.1% BSA. Expression of GFP-tagged proteins was directly visualized, while expression of MYC epitope-tagged proteins was visualized after a 60 minutes incubation with the 9E10 anti-MYC monoclonal antibody (gift from D. Mathieu, IGMM) (1:2 dilution in PBS/BSA), followed by incubation with affinity-purified fluorescein-conjugated goat anti-mouse antibody (Cappel-ICN) (1:40 dilution). Cells were simultaneously stained for F-actin using rhodamine-conjugated phalloidin (0.5 U.ml⁻¹; Sigma). Alternatively, cells were stained with an anti-ezrin polyclonal antibody, previously described (Andreoli et al., 1994) and provided by P. Mangeat, followed by incubation with affinity-purified fluorescein-conjugated goat anti-rabbit antibody (Cappel-ICN) (1:20 dilution). Cells were washed in PBS, mounted in Mowiol (Aldrich). To consider a cell having filopodia, we evaluated the number of filopodia on its surface: F-actin stained cells containing at least 5 filopodia were scored as being positive.

### Cell imaging.

For immunofluorescence, cells were observed using a DMR B microscope (Leica, Germany) with a PL APO 40 x objective (NA 1.00), or (for specification see figure legends) a PL APO 63, immersion oil Immersol 518 F (Zeiss, Germany) and illumination of the preparation by a 100 W HBO 103W/2 light bulb (OSRAM, Germany). Images thus obtained were captured with an ORCA 100 (B/W) 10 bits cooled CCD camera (C mount 1x), C 4742-95 controller and HIPIC controller program run by a PC compatible microcomputer (Hamamatsu, Japan). Images were saved as TIFF format (8 bits) for processing and mounting with Microsoft PowerPoint.

### SEM.

MEF and MEF p53-/- were grown on coverslips and then fixed in 0.1 M sodium cacodylate (pH 7.2) containing 2% glutaraldehyde and 0.1 M sucrose for at least 1h and processed as described (Brunk et al., 1981). Samples were observed using a Hitachi S4000 scanning microscope at 15kV.

### Time-lapse imaging.

Time-lapse phase contrast microscopy was performed on a Leica DL IRBE (Leica, Wetzlar, Germany) inverted microscope equipped with an automatic shutter and GFP filter sets, a 63X oil-immersion objective (NA1.3, Leica) sample heater (37°C) and a home-made CO₂ incubation chamber. Images were captured with a MicroMax 1300 CCD camera (RS-Princeton Instruments, Treuton, PA, USA) imaging software, converted to TIF files that were edited with NIH Image and compiled into QuickTime movies. The exposure time was fixed to 50 milliseconds.

### Cdc42 activity assay.

The Cdc42 activity assay was performed as described (Ory et al., 2000). Briefly, 3.10⁵ cells, transfected or not with p53 wt, were lysed before incubation with GST-PAK fusion protein the Cdc42-binding domain (CRIB) from human PAK1B (amino acids 56-272) coupled to glutathione-Sepharose beads (Pharmacia Biotech). After precipitation, complexes were washed four times with lysis buffer, eluted in SDS-PAGE sample buffer, immunoblotted and analysed with antibodies against Cdc42 (Transduction Laboratories). Aliquots taken from supernatants prior to precipitation were used to quantify total Cdc42 GTPase present in cell lysates.

### p53 transactivation assay.

p53 transactivation was measured using the dual-luciferase assay system from Promega. Cells (5x10⁴) were seeded onto twelve-well plates and transfected 18 hours later in OptiMEM containing 0.440 µg of DNA (0.2 µg of appropriate GTPase plasmids, 0.2 µg of pGL2B-mdm2-luciferase plasmid and 0.04 µg of pTKRL plasmid) using 0.33 µl of Lipofectamine (Gibco-BRL) for 4 h. Cells were then left for 24 h in DMEM supplemented with 10% FCS, harvested in 250 ml of Passive Lysis Buffer (PLB, Promega), and luciferase activity was measured following the Dual-luciferase^{™} Reporter Assay Protocol as recommended by Promega, using a luminometer fitted with two injectors (Berthold).

### EXAMPLE 1 p53 selectively inhibits morphological changes induced by Cdc42, but not those induced by Rac1 and RhoA.

To examine the overall effect of p53 on Rho GTPases-dependent F-actin structures, mouse embryonic fibroblasts (MEF) were transiently co-transfected with plasmids encoding wt p53 and green fluorescent protein (GFP)-tagged constitutively active forms of Cdc42 (Cdc42-V12), Rac1 (Rac1-V12) or RhoA (RhoA-V14). 20-24 hours later, the transfected cells were detected by fluorescence and analyzed for morphological changes associated with actin polymerization using rhodamine-labelled phalloidin. As shown in figure 1A, expression of GFP-tagged Cdc42-V12 alone led to the appearance of numerous long F-actin rich membrane extensions, called filopodia (panels a, b and c, arrows). In contrast, cells co-expressing p53 and GFP-Cdc42-V12 did not present any filopodia (panels a, b and c, arrowheads), suggesting that p53 inhibited Cdc42-V12 induced filopodia formation. Conversely, p53 expression had no effect on RhoA-dependent stress fibre formation (panels d, e and f), or on Rac1-dependent ruffles and lamellipodia (panels g, h and i). To confirm that the inhibition of Cdc42-induced filopodia was due to p53 activity, we performed cotransfection of the GFP-tagged Cdc42-V12 with two naturally occurring p53 mutants, namely p53 H273 and p53 H175 (Figure 1B). Both mutations map to the DNA binding domain of p53 and result in the loss of the sequence specific DNA binding activity, therefore acting as dominant negative mutants of the endogenous p53 (Ory et al., 1994). Expression of either p53 H273 (panel a) or p53 H175 (panel d) mutant had no effect on filopodia extensions (panels c and f) induced by Cdc42-V12 (panels b and e). A quantitative analysis shows that almost all Cdc42-V12-expressing cells had exaggerated filopodia (98 +/- 2 %, figure 1C). This number was reduced to 22 +/- 6% by the co-expression of p53 wt, but was unaffected by the co-expression of either of the two p53 mutants.

To overcome the possibility that suppression of filopodia was due to overexpression of ectopic p53 or Cdc42, an alternative approach was used; endogenous Cdc42 and p53 activities were stimulated (Figure 2). Cdc42-dependent filopodia formation was induced by bradykinin (Kozma et al., 1995; Nobes and Hall, 1995a). p53 was activated either by Tumor Necrosis Factor-a (TNFa), or using two cytotoxic drugs adriamycin and etoposide, previously shown to induce accumulation of p53 in MEF (Klefstrom et al., 1997; Lowe et al., 1993). First, we have verified that TNF a, adriamycin and etoposide upregulate endogenous p53 activity in MEF, by monitoring the p53 dependent transactivation of the firefly luciferase reporter gene linked to the p53 responsive promoter derived from the mdm2 gene (Barak et al., 1994)(Figure 2A). Expression of wt p53 led to an eighty-fold transactivation of the p53-responsive element. In contrast, expression of mutants p53 did not activate the mdm2 promoter. Treatment of cells with TNFα, adriamycin or etoposide resulted respectively in a twenty eight-fold, nineteen-fold and twenty fourfold activation of the p53-responsive element, reaching up to 36, 23 and 30 % of the value obtained with the positive control (p53 wt), respectively. Second, we controlled that bradykinin-induced filopodia formation in MEF cells depends on Cdc42 function; Expression of the dominant negative Cdc42 mutant (Cdc42-N17) abolished bradykinin induced filopodia in MEF (data not shown), as previously reported in other cell type (Kozma et al., 1995). Finally, we monitored the effect of p53 upregulation by TNFα, etoposide and adriamycin on Cdc42-dependent filopodia formation by bradykinin. As shown in figure 2B, bradykinin led to appearance of filopodia after 12 minutes of treatment (panel b). In contrast, pretreatment with TNFα, adriamycin or etoposide prior to bradykinin addition led to a dramatic decrease of filopodia formation (panels c, d and e, respectively). Quantification of these results (figure 2C) shows that bradykinin-mediated filopodia formation was reduced from 62 % to 28, 16 and 20 % by TNFα, adriamycin and etoposide, respectively. In addition, filopodia formation naturally occuring in 13 % of MEF was also reduced by endogenous p53 induction using either of these three drugs.

Taken together, these results indicate that both ectopic expression of p53 and activation of endogenous p53 lead to inhibition of Cdc42-induced filopodia formation.

### EXAMPLE 2 : p53-deficient fibroblasts accumulate filopodia.

The above results demonstrate that p53 interferes with the signal transduction pathway leading from activated Cdc42 to filopodia formation. The question arises as to whether the inactivation of p53 functions might affect actin cytoskeleton. To address this point, we analyzed F-actin organization of embryonic fibroblasts derived from mice harboring a targeted disruption of the p53 gene (MEF p53-/-). MEF p53 +/+ and MEF p53 -/- were taken at the same low passage number (1-6) to avoid genetic abnormalities acquired during continuous passage (Harvey et al., 1993). F-actin organization of these two cell types were compared using rhodamine-labeled phalloidin staining (figure 3A, panels a and b). Plasma membrane organization was also analysed by scanning electron microscopy (SEM) (panels c and d). Both methods pointed out a clear difference between the two cell types: MEF p53 -/- exhibit numerous F-actin-containing peripheral microspikes (arrows in panels b and d), whereas these structures are scarce in MEF p53 +/+ (panels a and c). In addition, globular membrane protrusions (arrowheads), only visible by SEM and not by F-actin staining, were exclusively observed in MEF p53 -/- (panel d). Finally, we stained MEF and MEF p53-/- with antibodies against ezrin, a marker of microspikes (Amieva et al., 1999). In normal MEF (panel e) ezrin was distributed throughout the cytoplasm, whereas a plasma localization both in thin membrane extensions (arrows) and in globular membrane protrusions (arrowheads) was observed in MEF p53-/- (panel f).

To ascertain that actin microspikes observed in MEF p53 -/resulted from membrane protrusions (filopodia) and not from membrane retractions (retractions fibers), we compared MEF p53 +/+ and MEF p53 -/- by time-lapse phase-contrast microscopy analysis. Time-lapse sequences were collected by taking one image every 3 seconds during 10 minutes. As shown in figure 3B and the accompanying videos, membranes of MEF p53 -/- display extensive dynamic activities and present a lot of extensions protruding out of the cell from peripheral globular membrane structures, rapidly extending and shortening (panel a). In contrast, membranes of MEF present much less protrusions, eliciting only large lamellipodia (panel b). To definitively ascertain the presence of filopodia in MEF p53 -/-, we performed high resolution phase contrast analysis (figure 3C) and identified these peripheral globular membrane structures as characteristics of the different stages of filopodia emergence as previously described (Steketee et al., 2001): an engorgement (E) consisting in an influx of cytoplasm into an associated adjacent filopodium, a development of a focal phase density (fd) at the leading margin or/and along the parental filopodium, and next protrusion of a convex projection with wide bases, namely nub (nb), that subsequently grew up and transformed into one or several filopodia, whose size (1-15 µm), morphology and dynamics are similar to those previously described (Kozma et al., 1995; Nobes and Hall, 1995b). Taken together these data demonstrate that MEF p53-/- are constitutively endowed with filopodia.

### EXAMPLE 3 : wild type p53 activity is required to inhibit filopodia formation.

In order to confirm the role of p53 in inhibition of filopodia formation, we next examined whether expression of wt p53 in MEF p53 - /- abolishes the constitutive presence of filopodia. F-actin organisation of MEF p53 -/- expressing either GFP-tagged p53 wt, p53 H273 or p53 H175 was analysed (figure 4A). Reintroduction of p53 wt in MEF p53 -/(panel a) prevented filopodia formation (panel b), whereas either of the two p53 mutants p53 H273 (panel c) or p53 H175 (panel e) were ineffective (panels d and f, respectively). GFP alone had no effect on filopodia formation in MEF p53 -/- (data not shown).

To examine the mechanism whereby p53 expression reverts filopodia formation in MEF p53 -/-, transfected cells were observed by phase contrast time-lapse microscopy. 12 hours after transfection, a microscope field with GFP-positive cells was selected. As shown in figure 4B and the accompanying videos, GFP-tagged p53 expression (panel a) restored a phenotype identical to MEF p53 +/+, i.e. protrusive structures on cell surface were barely detectable, signing the scarcity or the absence of filopodia (panel b). In contrast, under the same conditions, expression of the either of the two p53 mutants p53 H273 (panel c) or p53 H175 (not shown) had no obvious effect (panel d). Filopodia formation MEF p53 -/- expressing only GFP was not impaired (not shown). Quantitative analysis performed on numerous MEF p53 -/shows that the number of cells harboring filopodia was dramatically reduced by expression of p53 wt reintroduction (from 88 +/- 5 % to 22 +/-6 %), but not by the expression of the mutated p53 or the GFP alone (figure 4C).

All these experiments demonstrate that i/ p53 wt activity is required to inhibit filopodia, probably through its DNA binding and transcriptional activity; ii/ p53 inhibits initiating steps of filopodia formation, preventing focal densities and nubs appearance.

### EXAMPLE 4 : p53 acts downstream of Cdc42 to inhibit filopodia formation.

In order to further analyze the hierarchical organization of Cdc42 and p53 signaling in filopodia formation, we expressed GFP-tagged Cdc42-N17, the dominant negative form of Cdc42, in MEF p53-/- and analyzed the resulting F-actin modifications (figure 5A). Expression of Cdc42-N17 in MEF p53-/- (panel a) did not impair filopodia formation (panel b). Alternatively, inhibition of endogenous Cdc42 was performed by expression the Cdc42-interacting domain of the Wiskott-Aldrich syndrome protein (WASP), known to inhibit the endogenous Cdc42 activity through competition with its effector binding site (Aspenstrom et al., 1996). Expression of the WASP fragment (panel c) in MEF p53 -/- did not affect the abundance of filopodia (panel d). A quantitative analysis of these results confirmed that neither Cdc42-N17, nor the WASP fragment affect the number of MEF p53 -/- with filopodia (88 +/- 5% in control MEF p53 -/-; 82 +/- 4 % in Cdc42-N17-expressing MEF p53 -/-; and 86 +/- 6 % in WASP fragment-expressing MEF p53 -/-). Interestingly, the level of active Cdc42 was the same either in MEF, MEF p53-/- or MEF p53-/- expressing p53 wt, as tested by measuring the level of GTP-bound Cdc42 (Figure 5B). These data demonstrate that p53-dependent inhibition of filopodia does not occur at the Cdc42 level.

In order to test whether Cdc42 can rescue p53-dependent inhibition of filopodia formation in MEF p53 -/-, we co-transfected p53 wt with GFP-tagged Cdc42-V12 in MEF p53-/- (Figure 5C). Cells expressing both p53 wt (panel a) and Cdc42-V12 (panel b) still did not present any filopodia at their surface, although they feature other characteristics of Cdc42 activation, including reduction of stress fibers and increase in diffuse and punctuate actin staining (panel c). Taken together, these data strongly suggest that p53 acts downstream of Cdc42 to inhibit filopodia formation in MEF p53-/-.

### EXAMPLE 5: Analysis of the frequency value of filopodia generation in normal non cancerous cells, in non metastatic cancerous cells and in metastatic cancerous cells of various cell types.

### A. MATERIAL AND METHODS

### Cell lines

**CCD 18 Co :** Human cell line derived from normal colon, described by Sugarman BJ et al. Recombinant human tumor necrosis factor-alpha: effects on proliferation of normal and transformed cells in vitro. Science 230: 943-945, 1985.
**CCD 33Co** : Human adherent fibroblast cell line derived from normal colon.
**CCD 112Co** : Human adherent fibroblast cell line derived from normal colon.
**CCD 841 CoN** : Human adherent epithelial cell line derived from normal colon, described in J. Tissue Culture Methods 9: 117-122, 1985.
**FHC** : Human adherent epithelial cell derived from normal colon, decribed by Siddiqui KM and Chopra DP. Primary and long term epithelial cell cultures from human fetal normal colonic mucosa. In Vitro 20: 859-868, 1984.
**HCT 116** : Human epithelial cell line derived from colorectal carcinoma, described notably by Brattain MG et al. Heterogeneity of malignant cells from a human colonic carcinoma. Cancer Res. 41: 1751-1756, 1981
**HT 29** : Human epithelial cell line derived from colorectal adenocarcinoma, described notably by Hanski C et al. Tumorigenicity, mucin production and AM-3 epitope expression in clones selected from the HT-29 colon carcinoma cell line. Int. J. Cancer 50: 924-929, 1992
**LS 174 T** : Human epithelial cell line derived from colorectal adenocarcinoma, described notably by Tom BH et al. Human colonic adenocarcinoma cells. I. Establishment and description of a new line. In Vitro 12: 180-191, 1976.
**WiDr** : Human epithelial cell line derived from colorectal adenocarcinoma, described notably by Noguchi P et al. Characterization of WiDr: a human colon carcinoma cell line. In Vitro 15: 401-408, 1979
**SW 480** : Human epithelial cell line derived from colorectal adenocarcinoma, described notably by Schroy PC et al. Detection of p21 ras mutations in colorectal adenomas and carcinomas by enzyme-linked immunosorbent assay. Cancer 76: 201-209, 1995
**SW 620** : Human epithelial cell line derived from colorectal adenocarcinoma. Metastatic site : lymph node. SW620 was isolated from the tissue of a 51-year-old Caucasian male (blood group A, Rh+) as was SW480 (ATCC CCL-228).A recurrence of the malignancy resulted in a wide-spread metastasis from the colon to an abdominal mass. Descibed notably by Melcher R et al. Spectral karyotyping of the human colon cancer cell lines SW480 and SW620. Cytogenet. Cell Genet. 88: 145-152,2000
**SK-Co-1 :** Human epithelial cell line derived from colorectal adenocarcinoma. Metastatic site : ascites. Described notably by Pollack MS et al. HLA-A, B, C and DR alloantigen expression on forty-six cultured human tumor cell lines. J. Natl. Cancer Inst. 66: 1003-1012, 1981
**Colo 205** : : Human epithelial cell line derived from colorectal adenocarcinoma. Metastatic site : ascites. Described notably by Bjork P et al. Isolation, partial characterization, and molecular cloning of a human colon adenocarcinoma cell-surface glycoprotein recognized by the C215 mouse monoclonal antibody. J. Biol. Chem. 268: 24232-24241, 1993

### Time-lapse imaging and counting of filopodia dynamic.

Time-lapse phase contrast microscopy was performed on a Leica DL IRBE (Leica, Wetzlar, Germany) inverted microscope equipped with an automatic shutter, a 63X oil-immersion objective (NA1.3, Leica) sample heater (37°C) and a home-made CO₂ incubation chamber. Images were captured with a MicroMax 1300 CCD camera (RS-Princeton Instruments, Treuton, PA, USA) imaging software, converted to TIF files that were edited with NIH Image and compiled into QuickTime movies. The exposure time was fixed to 50 milliseconds.

To calculate the frequency of filopodia appearance, movies were analysed and the number of filopodia protuding out of one chosen cell in a constant unit of area (0,001 mm²) and time (1 min.) were counted. For each point approximatively 100 cells were examined. All assays were performed in triplicate.

### B. RESULTS

The results are shown in Table 1 below.

**Table 1 : Measure of the frequency of filopodia generation in normal and cancerous cells of different cell types.**

| Cell lines | ATCC Number | Morphology | Tissue | Frequency of filopodia (number.min^{- 1}/µm²) |
|---|---|---|---|---|
| Normal cells | | | | |
| | | | | |
| CCD 18Co | CRL 1459 | Fibroblast | Normal | 0.06 +/- 0.02 |
| CCD 33Co | CRL 1539 | Fibroblast | Normal | 0.05 +/- 0.01 |
| CCD 112Co | CRL 1541 | Fibroblast | Normal | 0.04 +/- 0.02 |
| CCD 841 CoN | CRL 1790 | Epithelial | Normal | 0.07 +/- 0.02 |
| FHC | CRL 1831 | Epithelial | Normal | 0.04 +/- 0.01 |
| Tumour cells | | | | |
| Non metastatic | | | | |
| HCT 116 | CCL 247 | Epithelial | carcinoma | 0.26 +/- 0.04 |
| HT 29 | HTB 38 | Epithelial | adenocarcinoma | 0.38 +/- 0.06 |
| LS 174T | CL 188 | Epithelial | adenocarcinoma | 0.34 +/- 0.03 |
| WiDr | CCL 218 | Epithelial | adenocarcinoma | 0.41 +/-0.05 |
| Metastatic | | | | |
| SW 480 | CCL 228 | Epithelial | adenocarcinoma | 0.61 +/- 0.05 |
| SW 620 | CCL 227 | Epithelial | AdenocarcinomaMetastatic site | 0.68 +/- 0.05 |
| SK-Co-1 | HTB 39 | Epithelial | AdenocarcinomaMetastatic site | 0.78 +/- 0.08 |
| Colo 205 | CCL 222 | Epithelial | AdenocarcinomaMetastatic site | 0.71 +/- 0.05 |

As shown in Table 1 above, the frequency values of filopodia generation or formation in the cells contained in the various cell populations studied allows to discriminate between, respectively, (i) normal non cancerous cells and cancerous cells and (ii) non metastatic cancerous cells and metastatic cancerous cells.

Taking into account the frequency values found for each class of cells, respectively 5 normal colon cell lines and 8 colon cancer cell lines, threshold frequency values can be determined which define the expected frequency of filopodia generation in (i) a control sample containing normal non cancerous cells and (ii) a control sample containing tumour cells, at least for epithelial cells, and more specifically epithelial cells derived from colon.
**A** : Cells contained in a sample will be classified as "normal non cancerous cells" for a frequency value of filopodia generation of less than 0.1 filopodia . min⁻¹/µm².
**B** : Cells contained in a sample will be classified as "cancerous cells" for a frequency value of filopodia generation of more than 0.1 filopodia . min⁻¹/µm².
**C** : Cells contained in a sample will be classified as "non metastatic cancerous cells" for a frequency value of filopodia generation of more than 0.1 and less than 0.5 filopodia . min⁻¹/µm².
**D** : Cells contained in a sample will be classified as "metastatic cancerous cells" for a frequency value of filopodia generation of more than 0.5 filopodia . min⁻¹/µm².

Indeed, The threshold frequency values of filopodia generation which have been defined above for cells derived from colon may somewhat vary if other cell types are considered.
However, the whole teachings of the present Patent Application fully enables the one skilled in the art to determine more accurate threshold frequency values for every cell type of interest, by assaying publicly available normal and cancerous cell lines, of every cell type of interest, before assaying unknown samples, according to the Material and Methods detailed in the Examples.

### REFERENCES

Adams, A.E., Johnson, D.I., Longnecker, R.M., Sloat, B.F. and Pringle, J.R. (1990) CDC42 and CDC43, two additional genes involved in budding and the establishment of cell polarity in the yeast Saccharomyces cerevisiae. J Cell Biol, 111, 131-42.
Aepfelbacher, M., Essler, M., Huber, E., Czech, A. and Weber, P.C. (1996) Rho is a negative regulator of human monocyte spreading. J Immunol, 157, 5070-5.
Aepfelbacher, M., Vauti, F., Weber, P.C. and Glomset, J.A. (1994) Spreading of differentiating human monocytes is associated with a major increase in membrane-bound CDC42. Proc Natl Acad Sci U S A, 91, 4263-7.
Agarwal, M.L., Taylor, W.R., Chernov, M.V., Chernova, O.B. and Stark, G.R. (1998) The p53 network. J Biol Chem, 273, 1-4.
Amieva, M.R., Litman, P., Huang, L., Ichimaru, E. and Furthmayr, H. (1999) Disruption of dynamic cell surface architecture of NIH3T3 fibroblasts by the N-terminal domains of moesin and ezrin: in vivo imaging with GFP fusion proteins. J Cell Sci, 112, 111-25.
Andreoli, C., Martin, M., Le Borgne, R., Reggio, H. and Mangeat, P. (1994) Ezrin has properties to self-associate at the plasma membrane. J Cell Sci, 107, 2509-21.
Aspenstrom, P., Lindberg, U. and Hall, A. (1996) Two GTPases, Cdc42 and Rac, bind directly to a protein implicated in the immunodeficiency disorder Wiskott-Aldrich syndrome. Curr Biol, 6, 70-5.
Bar-Sagi, D. and Feramisco, J.R. (1986) Induction of membrane ruffling and fluid-phase pinocytosis in quiescent fibroblasts by ras proteins. Science, 233, 1061-8.
Barak, Y., Lupo, A., Zauberman, A., Juven, T., Aloni-Grinstein, R., Gottlieb, E., Rotter, V. and Oren, M. (1994) Targets for transcriptional activation by wild-type p53: endogenous retroviral LTR, immunoglobulin-like promoter, and an internal promoter of the mdm2 gene. Cold Spring Harb Symp Quant Biol, 59, 225-35.
Braga, V.M., Machesky, L.M., Hall, A. and Hotchin, N.A. (1997) The small GTPases Rho and Rac are required for the establishment of cadherin-dependent cell-cell contacts. J Cell Biol, 137, 1421-31.
Brunk, U., Collins, V.P. and Arro, E. (1981) The fixation, dehydration, drying and coating of cultured cells of SEM. J Microsc, 123, 121-31.
Comer, K.A., Dennis, P.A., Armstrong, L., Catino, J.J., Kastan, M.B. and Kumar, C.C. (1998) Human smooth muscle alpha-actin gene is a transcriptional target of the p53 tumor suppressor protein. Oncogene, 16, 1299-308.
Dutartre, H., Davoust, J., Gorvel, J.P. and Chavrier, P. (1996) Cytokinesis arrest and redistribution of actin-cytoskeleton regulatory components in cells expressing the Rho GTPase CDC42Hs. J Cell Sci, 109, 367-77.
Eliyahu, D., Michalovitz, D., Eliyahu, S., Pinhasi-Kimhi, O. and Oren, M. (1989) Wild-type p53 can inhibit oncogene-mediated focus formation. Proc Natl Acad Sci U S A, 86, 8763-7.
Etienne-Manneville, S. and Hall, A. (2001) Integrin-mediated activation of Cdc42 controls cell polarity in migrating astrocytes through PKCzeta. Cell, 106, 489-98.
Gauthier-Rouviere, C., Vignal, E., Meriane, M., Roux, P., Montcourier, P. and Fort, P. (1998) RhoG GTPase controls a pathway that independently activates Rac1 and Cdc42Hs. Mol Biol Cell, 9, 1379-94.
Giaccia, A.J. and Kastan, M.B. (1998) The complexity of p53 modulation: emerging patterns from divergent signals. Genes Dev, 12, 2973-83.
Gloushankova, N., Ossovskaya, V., Vasiliev, J., Chumakov, P. and Kopnin, B. (1997) Changes in p53 expression can modify cell shape of ras-transformed fibroblasts and epitheliocytes. Oncogene, 15, 2985-9.
Guenal, I., Risler, Y. and Mignotte, B. (1997) Down-regulation of actin genes precedes microfilament network disruption and actin cleavage during p53-mediated apoptosis. J Cell Sci, 110, 489-95.
Hall, A. (1998) Rho GTPases and the actin cytoskeleton. Science, 279, 509-14.
Hansen, R. and Oren, M. (1997) p53; from inductive signal to cellular effect. Curr Opin Genet Dev, 7, 46-51.
Harvey, M., Sands, A.T., Weiss, R.S., Hegi, M.E., Wiseman, R.W., Pantazis, P., Giovanella, B.C., Tainsky, M.A., Bradley, A. and Donehower, L.A. (1993) In vitro growth characteristics of embryo fibroblasts isolated from p53- deficient mice. Oncogene, 8, 2457-67.
Hollstein, M., Rice, K., Greenblatt, M.S., Soussi, T., Fuchs, R., Sorlie, T., Hovig, E., Smith-Sorensen, B., Montesano, R. and Harris, C.C. (1994) Database of p53 gene somatic mutations in human tumors and cell lines. Nucleic Acids Res, 22, 3551-5.
Izawa, I., Amano, M., Chihara, K., Yamamoto, T. and Kaibuchi, K. (1998) Possible involvement of the inactivation of the Rho-Rho-kinase pathway in oncogenic Ras-induced transformation. Oncogene, 17, 2863-71.
Jacks, T., Remington, L., Williams, B.O., Schmitt, E.M., Halachmi, S., Bronson, R.T. and Weinberg, R.A. (1994) Tumor spectrum analysis in p53-mutant mice. Curr Biol, 4, 1-7.
Klefstrom, J., Arighi, E., Littlewood, T., Jaattela, M., Saksela, E., Evan, G.I. and Alitalo, K. (1997) Induction of TNF-sensitive cellular phenotype by c-Myc involves p53 and impaired NF-kappaB activation. Embo J, 16, 7382-92.
Kozma, R., Ahmed, S., Best, A. and Lim, L. (1995) The Ras-related protein Cdc42Hs and bradykinin promote formation of peripheral actin microspikes and filopodia in Swiss 3T3 fibroblasts. Mol Cell Biol, 15, 1942-52.
Levine, A.J. (1997) p53, the cellular gatekeeper for growth and division. Cell, 88, 323-31.
Lowe, S.W., Ruley, H.E., Jacks, T. and Housman, D.E. (1993) p53-dependent apoptosis modulates the cytotoxicity of anticancer agents. Cell, 74, 957-67.
Metcalfe, S., Weeds, A., Okorokov, A.L., Milner, J., Cockman, M. and Pope, B. (1999) Wild-type p53 protein shows calcium-dependent binding to F-actin. Oncogene, 18, 2351-5.
Nobes, C.D. and Hall, A. (1995a) Rho, rac, and cdc42 GTPases regulate the assembly of multimolecular focal complexes associated with actin stress fibers, lamellipodia, and filopodia. Cell, 81, 53-62.
Nobes, C.D. and Hall, A. (1995b) Rho, rac and cdc42 GTPases: regulators of actin structures, cell adhesion and motility. Biochem Soc Trans, 23, 456-9.
Nobes, C.D. and Hall, A. (1999) Rho GTPases control polarity, protrusion, and adhesion during cell movement. J Cell Biol, 144, 1235-44.
Ory, K., Legros, Y., Auguin, C. and Soussi, T. (1994) Analysis of the most representative tumour-derived p53 mutants reveals that changes in protein conformation are not correlated with loss of transactivation or inhibition of cell proliferation. Embo J, 13, 3496-504.
Ory, S., Munari-Silem, Y., Fort, P. and Jurdic, P. (2000) &Rgr; and rac exert antagonistic functions on spreading of macrophage-derived multinucleated cells and are not required for actin fiber formation [In Process Citation]. J Cell Sci, 113, 1177-88.
Philips, A., Roux, P., Coulon, V., Bellanger, J.M., Vie, A., Vignais, M.L. and Blanchard, J.M. (2000) Differential effect of rac and cdc42 on p38 kinase activity and cell cycle progression of nonadherent primary mouse fibroblasts [In Process Citation]. J Biol Chem, 275, 5911-7.
Price, L.S., Leng, J., Schwartz, M.A. and Bokoch, G.M. (1998) Activation of Rac and Cdc42 by integrins mediates cell spreading. Mol Biol Cell, 9, 1863-71.
Prives, C. (1998) Signaling to p53: breaking the MDM2-p53 circuit. Cell, 95, 5-8.
Qiu, R.G., Abo, A., McCormick, F. and Symons, M. (1997) Cdc42 regulates anchorage-independent growth and is necessary for Ras transformation. Mol Cell Biol, 17, 3449-58.
Roux, P., Gauthier-Rouviere, C., Doucet-Brutin, S. and Fort, P. (1997) The small GTPases Cdc42Hs, Rac1 and RhoG delineate Raf-independent pathways that cooperate to transform NIH3T3 cells. Curr Biol, 7, 629-37.
Steketee, M., Balazovich, K. and Tosney, K.W. (2001) Filopodial initiation and a novel filament-organizing center, the focal ring. Mol Biol Cell, 12, 2378-95.
Tapon, N. and Hall, A. (1997) Rho, Rac and Cdc42 GTPases regulate the organization of the actin cytoskeleton. Curr Opin Cell Biol, 9, 86-92.
Zohn, I.M., Campbell, S.L., Khosravi-Far, R., Rossman, K.L. and Der, C.J. (1998) Rho family proteins and Ras transformation: the RHOad less traveled gets congested. Oncogene, 17, 1415-38.

## Claims

1. Use of the frequency value of filopodia generation in a sample cell population as a biological marker for determining the cancerous, metastatic cancerous or normal phenotype of said cell population, wherein the frequency of filopodia generation consists of counting the number of filopodia formed at successive instants during a predetermined period of time.

2. A method for determining *in vitro* the cancerous, metastatic cancerous or normal phenotype of cells contained in a sample, wherein said method comprises a step of *in vitro* measuring the frequency of filopodia generation in the cells contained in the sample, wherein the frequency of filopodia generation consists of counting the number of filopodia formed at successive instants during a predetermined period of time.

3. The method according to claim 2 for determining *in vitro* the cancerous or normal phenotype of cells contained in a sample comprising the steps of:
a) measuring the frequency of filopodia generation in the cells contained in the sample;
b) comparing the frequency measured in step a) with the expected frequency of filopodia generation in (i) a control sample containing normal non cancerous cells or (ii) a control sample containing tumour cells.

4. The method according to claim 2 for determining *in vitro* the metastatic cancerous or the cancerous phenotype of cells contained in a sample comprising the steps of:
a) measuring the frequency of filopodia generation in the cells contained in the sample;
b) comparing the frequency measured in step a) with the expected frequency of filopodia generation in (i) a control sample containing cancerous non metastatic cells or (ii) a control sample containing metastatic tumour cells.

5. The method according to any one of claims 2 and 3, wherein the frequency of filopodia generation is measured by microscopy.

6. A method for the *in vitro* screening of the carcinogenic properties of a compound comprising the steps of :
a) incubating cells having a normal phenotype with said compound;
b) detecting the conversion of the normal cells into tumour cell phenotype by carrying out the method of claims 2, 3 and 5.

7. A method for the *in vitro* screening of the metastatic properties of a compound comprising the steps of:
a) incubating cells having a non metastatic tumour phenotype with said compound;
b) detecting the conversion of the tumour cells into metastatic tumour phenotype by carrying out the method of claims 2, 4 and 5.

8. A method for the *in vitro* screening of the anti-carcinogenic properties of a compound comprising the steps of :
a) incubating cells having a tumour cell phenotype with said compound;
b) detecting the conversion of the tumour cells into non-tumour cell phenotype by carrying out the method of claims 2, 3 and 5.

9. A method for the *in vitro* screening of the anti-metastatic properties of a compound comprising the steps of :
a) incubating cells having metastatic tumour cell phenotype with said compound;
b) detecting the conversion of the metastatic tumour cells into non-metastatic tumour cell phenotype or into non-tumour cell phenotype by carrying out the method of claims 2 to 5.

10. A method for designing an anti-cancerous therapeutic treatment of a cancerous patient comprising the step of determining the cancerous state of cells contained in a sample derived from a primary tumour of said patient by carrying out the method of any one of claims 2 to 5.

## Patentansprüche

1. Verwendung des Frequenzwerts der Filopodienbildung in einer Zellprobenpopulation als biologischen Marker zum Bestimmen des krebsartigen, des metastatisch krebsartigen oder des normalen Phänotyps der Zellpopulation, wobei die Frequenz der Filopodienbildung sich aus dem Zählen der Anzahl von Filopodien ergibt, welche zu aufeinanderfolgenden Zeitpunkten während eines vorbestimmten Zeitraums gebildet werden.

2. Verfahren zur *in-vitro*-Bestimmung des krebsartigen, des metastatisch krebsartigen oder des normalen Phänotyps von in einer Probe enthaltenen Zellen, wobei das Verfahren den Schritt des *in-vitro*-Messens der Frequenz der Filopodienbildung in den in der Probe enthaltenen Zellen, wobei die Frequenz der Filopodienbildung sich aus dem Zählen der Anzahl von Filopodien ergibt, welche zu aufeinanderfolgenden Zeitpunkten während eines vorbestimmten Zeitraums gebildet werden.

3. Verfahren nach Anspruch 2 zur *in-vitro*-Bestimmung des metastatischkrebsartigen oder des krebsartigen Phänotyps von in einer Probe enthaltenen Zellen, mit den folgenden Schritten:
a) Messen der Frequenz der Filopodienbildung in den in der Probe enthaltenen Zellen;
b) Vergleichen der im Schritt a) gemessenen Frequenz mit der erwarteten Frequenz der Filopodienbildung in (i) einer normale, nicht krebsartige Zellen enthaltenden Kontrollprobe oder (ii) einer Tumorzellen enthaltenden Kontrollprobe.

4. Verfahren nach Anspruch 2 zur *in-vitro*-Bestimmung des krebsartigen oder des normalen Phänotyps von in einer Probe enthaltenen Zellen, mit den folgenden Schritten:
a) Messen der Frequenz der Filopodienbildung in den in der Probe enthaltenen Zellen;
b) Vergleichen der im Schritt a) gemessenen Frequenz mit der erwarteten Frequenz der Filopodienbildung in (i) einer krebsartige, nicht metastatische Zellen enthaltenden Kontrollprobe oder (ii) einer metastatische Tumorzellen enthaltenden Kontrollprobe.

5. Verfahren nach einem der Ansprüche 2 und 3, bei welchem die Frequenz der Filopodienbildung durch Mikroskopie gemessen wird.

6. Verfahren zum *in-vitro*-Screening der karzinogenen Eigenschaften einer Verbindung, mit den folgenden Schritten:
a) Inkubieren von einen normalen Phänotyp aufweisenden Zellen mit der Verbindung;
b) Erkennen der Umwandlung der normalen Zellen in einen Tumorzellen-Phänotyp durch das Durchführen des Verfahrens nach den Ansprüchen 2, 3 und 5.

7. Verfahren zum *in-vitro*-Screening der metastatischen Eigenschaften einer Verbindung, mit den folgenden Schritten:
a) Inkubieren von einen nicht-Verbindung Tumor-Phänotyp aufweisenden Zellen mit der Verbindung;
b) Erkennen der Umwandlung der Tumorzellen in einen metastatischen Tumorzellen-Phänotyp durch das Durchführen des Verfahrens nach den Ansprüchen 2, 4 und 5.

8. Verfahren zum *in-vitro*-Screening der antikarzinogenen Eigenschaften einer Verbindung, mit den folgenden Schritten:
a) Inkubieren von einen Tumorzellen-Phänotyp aufweisenden Zellen mit der Verbindung;
b) Erkennen der Umwandlung der Tumorzellen in einen Nicht-Tumorzellen-Phänotyp durch das Durchführen des Verfahrens nach den Ansprüchen 2, 3 und 5.

9. Verfahren zum *in-vitro*-Screening der antimetastatischen Eigenschaften einer Verbindung, mit den folgenden Schritten:
a) Inkubieren von einen metastatischen Tumorzellen-Phänotyp aufweisenden Zellen mit der Verbindung;
b) Erkennen der Umwandlung der metastatischen Tumorzellen in einen nicht-metastatischen Tumorzellen-Phänotyp durch das Durchführen des Verfahrens nach den Ansprüchen 2 bis 5.

10. Verfahren zum Entwickeln einer therapeutischen Krebsbehandlung eines Krebspatienten mit dem Schritt des Bestimmens des Krebszustands von Zellen in einer aus einem primären Tumor des Patienten entnommenen Probe durch das Durchführen des Verfahrens nach einem der Ansprüche 2 bis 5.

## Revendications

1. Utilisation de la valeur de la fréquence de production de filopodes dans une population cellulaire d'échantillon, comme marqueur biologique pour la détermination du phénotype cancéreux, cancéreux métastatique ou normal de ladite population cellulaire, où la fréquence de production de filopodes consiste à compter le nombre de filopodes formés à des instants successifs pendant une période de temps prédéterminée.

2. Procédé pour déterminer in vitro le phénotype cancéreux, cancéreux métastatique ou normal des cellules présentes dans un échantillon, où ledit procédé comprend une étape de mesure in vitro de la fréquence de production de filopodes dans les cellules présentes dans l'échantillon, dans lequel la fréquence de production de filopodes consiste à compter le nombre de filopodes formés à des instants successifs pendant une période de temps prédéterminée.

3. Procédé selon la revendication 2 pour déterminer in vitro le phénotype cancéreux ou normal des cellules présentes dans un échantillon, comprenant les étapes consistant à:
a) mesurer la fréquence de production de filopodes dans les cellules présentes dans l'échantillon;
b) comparer la fréquence mesurée dans l'étape a) à la fréquence prévue de production de filopodes dans (i) un échantillon témoin contenant des cellules normales non cancéreuses ou (ii) un échantillon témoin contenant des cellules tumorales.

4. Procédé selon la revendication 2 pour déterminer in vitro le phénotype cancéreux métastatique ou cancéreux des cellules présentes dans un échantillon, comprenant les étapes consistant à:
a) mesurer la fréquence de production de filopodes dans les cellules présentes dans l'échantillon;
b) comparer la fréquence mesurée dans l'étape a) à la fréquence prévue de production de filopodes dans (i) un échantillon témoin contenant des cellules cancéreuses non métastatiques ou (ii) un échantillon témoin contenant des cellules tumorales métastatiques.

5. Procédé selon l'une quelconque des revendications 2 et 3, dans lequel la fréquence de production de filopodes est mesurée par microscopie.

6. Procédé pour la recherche systématique in vitro des propriétés carcinogènes d'un composé, comprenant les étapes qui consistent à:
a) incuber des cellules ayant un phénotype normal avec ledit composé;
b) détecter la conversion des cellules normales en phénotype de cellules tumorales en mettant en oeuvre le procédé des revendications 2, 3 et 5.

7. Procédé pour la recherche systématique in vitro des propriétés métastatiques d'un composé, comprenant les étapes qui consistent à:
a) incuber les cellules ayant un phénotype de tumeur non métastatique avec ledit composé;
b) détecter la conversion des cellules tumorales en phénotype de tumeur métastatique en mettant en oeuvre le procédé des revendications 2, 4 et 5.

8. Procédé pour la recherche systématique in vitro des propriétés anti-carcinogènes d'un composé, comprenant les étapes qui consistent à:
a) incuber les cellules ayant un phénotype de cellules tumorales avec ledit composé;
b) détecter la conversion des cellules tumorales en phénotype de cellules non tumorales en mettant en oeuvre le procédé des revendications 2, 3 et 5.

9. Procédé pour la recherche systématique in vitro des propriétés anti-métastatiques d'un composé, comprenant les étapes qui consistent à:
a) incuber les cellules ayant un phénotype de cellules tumorales métastatiques avec ledit composé;
b) détecter la conversion des cellules tumorales métastatiques en phénotype de cellules tumorales non métastatiques ou en phénotype de cellules non tumorales en mettant en oeuvre le procédé des revendications 2 à 5.

10. Procédé pour concevoir un traitement thérapeutique anticancéreux d'un patient cancéreux, comprenant l'étape de détermination de l'état cancéreux des cellules présentes dans un échantillon provenant d'une tumeur primaire dudit patient en mettant en oeuvre le procédé de l'une quelconque des revendications 2 à 5.
